# EUROPÄISCHE PATENTSCHRIFT

(11) **EP 3 325 356 B1**
(45) Veröffentlichungstag und Bekanntmachung des Hinweises auf die Patenterteilung: **06.11.2019**
(21) Anmeldenummer: 16741305.3
(22) Anmeldetag: 20.07.2016
(51) Int. Cl.: B65B 55/10, B65B 5/06

(54) **VERFAHREN ZUM STERILISIEREN VON UNVERPACKTEN GEBRAUCHSGÜTERN SOWIE DAZU VERWENDBARE SAMMELBEHÄLTER UND UMVERPACKUNGEN**
METHOD FOR STERILIZING NON-PACKAGED ARTICLES OF USE, AND COLLECTING CONTAINERS AND EXTERNAL PACKAGINGS THAT CAN BE USED THEREFOR
PROCÉDÉ POUR STÉRILISER DES PRODUITS DE CONSOMMATION NON EMBALLÉS ET RÉCIPIENTS DE RECUEILS ET EMBALLAGES POUVANT ÊTRE UTILISÉS À CET EFFET

(30) Priorität: 23.07.2015 DE 102015112034; 30.09.2015 DE 102015116536
(43) Veröffentlichungstag der Anmeldung: 30.05.2018
(73) Patentinhaber: SIG Technology AG, 8212 Neuhausen am Rheinfall (CH)
(72) Erfinder: MAINZ, Hans-Willi, 52525 Heinsberg (DE); SCHREDER, Michael, 6395 Hochfilzen (AT)
(74) Vertreter: Cohausz & Florack
(86) Internationale Anmeldenummer: PCT/EP2016/067243
(87) Internationale Veröffentlichungsnummer: WO 2017/013150

(56) Entgegenhaltungen:
- DE-U1- 29 903 534
- JP-A- 2015 063 324

## Beschreibung

Die Erfindung betrifft zunächst ein Verfahren zum Sterilisieren von unverpackten Gebrauchsgütern, wie insbesondere aus Kunststoff bestehenden Öffnungs- und/oder Verschlusselementen zur späteren Verwendung mit Getränke- bzw. Lebensmittelpackungen mittels einem ein Entkeimungsmittel aufweisenden Behandlungsmittel, wobei eine bestimmte Menge an unverpackten Gebrauchsgütern (3) gemeinsam für eine vorgegebene Zeit in einem Sammelbehälter (1) gelagert und das Innere des Sammelbehälters (1) mit einer vorgegebenen Menge des Behandlungsmittels beladen wird. Darüber hinaus werden ein Sammelbehälter zur Aufnahme einer Vielzahl ungeordneter Gebrauchsgüter sowie eine Umverpackung zur Aufnahme eines oder mehrerer solcher Sammelbehälter/s beansprucht.

Die Art der zu sterilisierenden Gebrauchsgüter ist äußerst vielfältig. Insbesondere richtet sich die Erfindung auf solche Gebrauchsgüter, die Verpackungen oder Verpackungsteile für Lebensmittel oder den medizinischen Bereich betreffen. Nachfolgend wird die Erfindung beispielhaft anhand der Sterilisierung von insbesondere aus Kunststoff bestehenden Öffnungs- und/oder Verschlusselementen zur späteren Verwendung mit Getränke- bzw. Lebensmittelpackungen näher erläutert.

Wenn im Folgenden von Öffnungs- und/oder Verschlusselementen für Getränke- bzw. Lebensmittelpackungen die Rede ist, so sollen hierunter nicht nur die am meisten verbreiteten wiederverschließbaren Ausgießelemente fallen, sondern jegliche Art von Öffnungshilfen und/oder Verschlüssen für solche Behältnisse.

An die aseptische Abfüllung von Getränken bzw. Lebensmitteln werden seit jeher höchste Anforderungen hinsichtlich der Keimfreiheit gestellt. So ist es seit langem bekannt, die entsprechenden Packungen und auch zugehörige und (spätestens beim Öffnen) mit dem Produkt in Kontakt tretende zusätzliche Elemente wie Öffnungselemente, Verschlüsse oder Ausgießelemente zu sterilisieren, wobei die Sterilisation meist unmittelbar vor dem Füllvorgang und abschließendem Versiegeln der Packung stattfindet.

In der Regel erfolgt das Abfüllen auf sog. Füllmaschinen, welche beim Abfüllbetrieb stehen und die zu füllenden Behältnisse müssen von ihrem Herstellungsort zum Ort des Abfüllbetriebs transportiert werden.

Aus der WO2012/00573 A1 ist eine Vorrichtung zum Sterilisieren von Verschlüssen für Behälter bekannt, bei der eine Vielzahl von kappenartigen Verschlüssen durch mehrere Behandlungszonen transportiert und dort mittels Beaufschlagung von H₂O₂-Dampf sterilisiert werden. Hier erfolgt die Sterilisation durch unmittelbares Beaufschlagen der Öffnungs- und/oder Verschlusselemente mit Entkeimungsmittel und durch einen anschließenden Trocknungsvorgang, um das H₂O₂ zuverlässig wieder zu entfernen.

Aus der DE 40 31 472 A1 ist eine Vorrichtung zum Sterilisieren, Füllen und Verschließen von eine Füllöffnung aufweisenden Behältern für sich bekannt.

Die DE 197 27 942 A1 beschreibt eine Maschine und ein Verfahren zum Verschließen von Flaschen mit Verschlusskappen mit einer entsprechenden Sterilisationseinrichtung.

Ein Verfahren zum Sterilisieren von Verschlusskappen ist aus der JP 2015 063 324 A bekannt. Dieses Verfahren sieht vor, mehrere Verschlusskappen in einen Aufbewahrungsbehälter zu gegeben, diesen mit Wasserstoffperoxid Lösung zu beaufschlagen und anschließend abzudichten und eine vorbestimmte Zeit lang stehen zu lassen.

Meistens erfolgt die Sterilisation der Öffnungs- und/oder Verschlusselemente unmittelbar vor dem Aufbringen auf die zu befüllenden Behälter, wie es beispielsweise aus der DE 10 2005 032 322 A1 bekannt ist.

Da die gebräuchlichen Öffnungs- und/oder Verschlusselemente in der Regel spritzgegossen sind, erfolgt deren Herstellung unter Umständen an einem weiteren Ort, so dass auch diese Elemente zum Abfüllbetrieb transportiert werden müssen.

Nach der Herstellung und insbesondere auf dem Transportweg sind die vereinzelten Elemente dabei stets der Gefahr einer Verkeimung durch vegetative Mikroorganismen ausgesetzt.

Als vegetative Mikroorganismen werden durch Zellteilung vermehrungsfähige Einzeller verstanden, die geeignet sind sich im Füllgut (,Produkt') einer Packung zu vermehren und dabei die Eigenschaften des Füllgutes zu verändern. Weiter umfasst der Begriff auch die Überdauerungsformen der vermehrungsfähigen Einzeller, wie zum Beispiel deren Sporen.

Diese Sporen sind meist sehr resistent gegen Veränderungen der sie umgebenden Umweltbedingungen. Wenn Mikroorganismen keine Umgebung zum Stoffwechsel und/oder zur Fortpflanzung vorfinden, haben einige Mikroorganismen die Möglichkeit, in ein Sporenstadium überzugehen.

Genauer sollen unter dem Begriff Mikroorganismen im Sinne der vorliegenden Anmeldung Eukaryoten und Prokaryoten verstanden werden, wobei die Eukaryoten eine echte Zellwand aufweisen und Algen, Protozonen, Pilze und Schleimpilze umfassen, während die Prokaryoten die Gruppe der Bakterien abdecken (vgl. ,Bergey's Manual of Determinative Bacteriology', 8. Auflage, Baltimore: Williams & Wilkins, 1974).

Speziell bei den Prokaryoten sind Überdauerungsformen, wie beispielsweise Sporen, bekannt. Diese sind vermehrt beispielsweise auch nach thermischen und/oder chemischen Behandlungen von Rohstoffen zur Herstellung von Kartonroherzeugnissen in eben diesen anzutreffen, da derartige Behandlungsmethoden die direkt vermehrungsfähige Form der Mikroorganismen entweder abtötet oder den Übergang in die Sporenform initiiert.

Als Maß für die Anzahl beziehungsweise die Menge der in einer Stoffmenge, (beispielsweise in dem angesprochenen Kartonroherzeugnis) enthaltenen Mikroorganismen, ist dem Fachmann der Begriff der "koloniebildenden Einheit pro Gewichts- oder Flächeneinheit" (kbE/g) oder (kbE/cm²) bekannt. Im Unterschied zu der direkten Auszählung aller vorhandenen Mikroorganismen mit einem geeigneten optischen Mittel, erfolgt die Bestimmung der Anzahl der koloniebildenden Einheiten über die gezielte Vermehrung der vorhandenen teilungsfähigen Mikroorganismen unter geeigneten Anzuchtbedingungen. Im Allgemeinen geschieht dies bis zu einer Koloniegröße, die mit dem unbewehrten Auge zählbar ist. Dabei nutzt man die Tatsache, dass aus jedem einzelnen teilungsfähigen Mikroorganismus unter zuvor definierten Bedingungen genau eine Kolonie entsteht. Einzelfälle, in denen zwei kbE so nah zusammenliegen, dass sich nur eine sichtbare Kolonie aus ihnen bildet, werden dann regelmäßig vernachlässigt.

In der Mikrobiologie typische Bestimmungsverfahren sind in der ISO 8784-1 aus 2005 geregelt.

Eine Reduzierung der kbE/g bzw. kbE/cm² wird vom Fachmann demzufolge als Maß für die Wirksamkeit eines Verfahrens zur Keimreduktion verwendet und häufig Entkeimungsrate genannt. Daraus abgeleitet ergibt sich die über die Anzahl von produzierten Packungen zu zählende Sterilitätsrate. Für aseptisch verpackte Lebensmittel galt dabei häufig, dass maximal der Inhalt einer von 3.000 produzierten Packungen im Laufe des angegebenen Mindesthaltbarkeitsdatums von in der Regel bis zu 12 Monaten unsteril werden durfte. Jüngere Bestrebungen der Anmelderin zielen nun auf eine Sterilitätsrate von maximal einer Packung auf 30.000 oder sogar 50.000 produzierten Packungen.

Es ist aus der DE 10 2011 111 523 A1 auch bereits bekannt, oben bzw. unten offene Schnittkanten eines Packungsmantels eines Verpackungsmaterials durch Aufbringen eines Behandlungsmittels, welches ein Entkeimungsmittel enthält, zu behandeln, wobei das Entkeimungsmittel nach dem Aufbringen auf den Schnittkanten verbleibt und in das Verpackungsmaterial eindringt. Das Aufbringen geschieht dabei jeweils durch Besprühen von oben, wobei eine Mehrzahl von Packungsmänteln flachgefaltet zusammengefasst sind.

Versuche haben gezeigt, dass die Idee des längerfristigen Einwirkens von Behandlungsmittel auf den zu behandelten Gegenstand während der - ohnehin zur Verfügung stehenden - Transportzeit nicht nur bei fibrösem Verpackungsmaterial, sondern auch auf die glatten Oberflächen anderer unverpackter Gebrauchsgüter wie beispielsweise von Öffnungs- und/oder Verschlusselementen übertragbar ist. Dies ist insbesondere dann von erhöhtem Interesse, wenn die Öffnungs- und/oder Verschlusselemente nicht nur auf eine Verpackung appliziert werden sollen, sondern selbst einen Teil einer Packung bilden, beispielsweise den Kopfbereich einer Karton/Kunststoff-Verbundpackung, bei der die eigentliche Ausgießöffnung nur einen kleinen Teil des komplett aus Kunststoff gespritzten Kopfteiles ausmacht. Also jedenfalls in den Fällen, in denen Bereiche der Öffnungs- und/oder Verschlusselemente vor dem Öffnen der Packung mit dem in der Packung beinhalteten Produkt in Kontakt treten.

Aus der US 9 078 435 A1 ist nun ein Verfahren zur Sterilisation von unverpackten Behältern bekannt, bei dem die später hygienisch verwendeten Behälter durch Eingabe eines, ein Entkeimungsmittel und einen Reaktionsbeschleuniger aufweisendes, Behandlungsmittel in den Behälter eingegeben wird und dieser über einen längeren Zeitraum von mehreren Minuten bis mehreren Tagen fest verschlossen oder versiegelt wird und die inneren Wandflächen des Behälters dadurch desinfiziert werden. Weiter soll dadurch auch der den Behälter verschließende Verschluss desinfiziert werden. Die Lehre der US 9,078,435 A1 zielt dabei auf die Desinfektion so genannter "Bag-in-Box" - Tüten ab, die wegen ihrer späteren Verwendung sehr hohe Barriereeigenschaften haben müssen, also eine sehr geringe Durchlässigkeit gegen Wasser und Gas aufweisen müssen. Da der Behälter, im Einzelnen also die "Bag-in-Box" Tüte, zur späteren Zweckerfüllung jedoch nur eine geringe Menge an Behandlungsmittel aufweisen darf, wird in den offenbarten Verfahren mit einer äußerst geringen Konzentration an Entkeimungsmittel gearbeitet. Dabei ist die Verwendung äußerst geringer Konzentrationen des Entkeimungsmittels, vorzugsweise H₂O₂, auch durch die ansonsten entstehenden Schäden an den direkt mit dem Behandlungsmittel in Kontakt tretenden Behälterwänden notwendig.

Davon ausgehend liegt der vorliegenden Erfindung die Aufgabe zugrunde, das eingangs genannte und zuvor näher beschriebene Verfahren zum Sterilisieren von unverpackten Gebrauchsgütern, wie insbesondere aus Kunststoff bestehenden Öffnungs- und/oder Verschlusselementen zur späteren Verwendung mit Getränke- bzw. Lebensmittelpackungen, mittels einem ein Entkeimungsmittel aufweisenden Behandlungsmittel, wobei eine bestimmte Menge an unverpackten Gebrauchsgütern (3) gemeinsam für eine vorgegebene Zeit in einem Sammelbehälter (1) gelagert und das Innere des Sammelbehälters (1) mit einer vorgegebenen Menge des Behandlungsmittels beladen wird, so auszugestalten und weiterzubilden, dass eine Kontaminierung der Gebrauchsgüter mit keimbildenden Sporen zuverlässig ausgeschlossen wird. Insbesondere ist erwünscht, eine "Langzeitsterilisation" zu erreichen, bei der die Sterilisation der behandelten Gebrauchsgüter bereits vor deren endgültiger Verwendung erfolgt. Für das Beispiel der Öffnungs- und/oder Verschlusselemente bedeutet dies, dass deren Sterilisation schon vor dem Einschleusen der Öffnungs- und/oder Verschlusselemente in eine Füllmaschine abgeschlossen sein soll. Weist die Füllmaschine eine Aseptikzone auf, kann es innerhalb der Füllmaschine noch einmal zu einer Nachdesinfektion der Öffnungs- und/oder Verschlusselemente kommen.

Gelöst wird diese Aufgabe bei einem Verfahren mit den Merkmalen gemäß dem Oberbegriff von Anspruch 1 dadurch, ein Beladungsträger, welcher mit Sterilisationsmittel behandelt worden ist, in den Sammelbehälter eingebracht wird. Das Verfahren kann auch als ,direkte mittelbare Sterilisation' bezeichnet werden. Der Sammelbehälter selbst ist im Sinne der vorliegenden Schrift nicht als Gebrauchsgut anzusehen.

Ein entsprechender Sammelbehälter mit den Merkmalen des Oberbegriffs von Anspruch 14 löst die Aufgabe dadurch, dass ein Beladungsträger, der mit der ausreichenden Menge Sterilisationsmittel behandelt worden ist, in den Sammelbehälter eingebracht wird.

Eine dazu verwendbare Umverpackung gemäß dem Oberbegriff von Anspruch 19 ist zur Lösung der Aufgabe dadurch gekennzeichnet, dass der Umkarton in seinem Inneren teilweise mit Beladungsmittel benetzt wird oder ein Beladungsträger, der mit der ausreichenden Menge Sterilisationsmittel behandelt worden ist, in den Umkarton eingebracht wird. Damit erfolgt ein sicherer Transport der behandelten Gebrauchsgüter zum Einsatzort.

Nach einer weiteren Lehre der Erfindung erfolgt die Beladung der einzelnen Öffnungs- und/oder Verschlusselemente maschinell, wobei vorzugsweise eine Sprühdüse eingesetzt wird. Bevorzugt erfolgt ein Sprühen ohne Luft, wobei unmittelbar anschließend der Sammelbehälter zu verschließen ist. Es ist jedoch auch denkbar, dass die Beladung manuell erfolgen kann. In jedem Fall sollte die Beladung möglichst unmittelbar nach der Herstellung der Gebrauchsgüter, im Beispiel also nach dem sich an den Spritzgießvorgang anschließenden Abkühlen der Öffnungs- und/oder Verschlusselemente erfolgen, um eine Verkeimung von vornherein zu vermeiden.

Nach einer weiteren bevorzugten Lehre der Erfindung wird als Beladungsmittel Wasserstoffperoxid (H₂O₂) verwendet und beträgt die Konzentration des Behandlungsmittels zwischen 20 % und 50 %, vorzugsweise 35 %.

Eine andere bevorzugte Ausgestaltung sieht vor, dass die Menge der jeweiligen Beladung mit Beladungsmittel in Abhängigkeit vom Volumen und von der Größe bzw. Oberfläche der zu sterilisierenden Gebrauchsgüter zwischen 0,5 ml und 2 ml beträgt. Die tatsächlich benötigte Menge lässt sich dabei leicht durch Vergleichsversuche ermitteln.

Für das Bespiel der Öffnungs- und/oder Verschlusselemente wurde herausgefunden, dass eine Dosierung von einem Milliliter für die Sterilisierung von 1.200 Öffnungs- und/oder Verschlusselementen ausreicht. Diese "Standarddosierung" ist an das Volumenverhältnis der Standardkartongröße der Umverpackung, welche im Bespiel 56 Liter beträgt, angepasst. Der Gesamtanteil des Volumens der Öffnungs- und/oder Verschlusselemente beträgt dabei 14 Liter, so dass das maximale freie Luftvolumen 42 Liter beträgt. Unter "Standarddosierung" ist eine Normaldosierung gemeint, da ein Überdosierung zu einer Kondensatbildung im Sammelbehälter führt, was unbedingt zu vermeiden ist und eine Unterdosierung nicht ausreicht, alle im Sammelbehälter befindlichen Gebrauchsgüter zuverlässig zu sterilisieren. Es ist klar, dass bei abweichenden Volumina die einzusetzende Dosiermenge angepasst werden muss. Die Normaldosierung beträgt etwa 1ml/56 l. Eine Abweichung von + oder - 25% liegt aber noch im Rahmen der Normaldosierung.Der Volumenanteil der Gebrauchsgüter liegt dabei bei ¼ des vom Sammelbehälter bzw. des Umkartons eingeschlossenen Volumens.

Bevorzugt beträgt die Dauer der Aussetzung mit Beladungsmittel im Sammelbehälter zwischen 12 Stunden und 21 Tagen, besonders bevorzugt liegt sie zwischen 24 Stunden und 7 Tagen. Auf diese Weise ist gewährleistet, dass während der Transportzeit zwischen Hersteller und dem Eintreffen beim Kunden eine vollkommen Sterilisation stattgefunden hat, so dass die beispielsweise Öffnungs- und/oder Verschlusselemente mehr oder weniger unmittelbar nach dem Auspacken des Sammelbehälters der Aseptikzone einer Füllmaschine zugeführt werden können.

Bevorzugt ist das Verfahren sogar frei von einer aktiven Entfernung des Behandlungsmittels. Das bedeutet, dass das Behandlungsmittel nicht aktiv von den sterilisierten unverpackten Gebrauchsgütern, wie insbesondere aus Kunststoff bestehenden Öffnungs- und/oder Verschlusselementen zur späteren Verwendung mit Getränke- bzw. Lebensmittelpackungen, entfernt wird. Zwar ist die rückstandslose Entfernung von Entkeimungsmitteln an mit dem Produkt in Kontakt tretenden Bereichen einer Lebensmittelverpackung eine zu erfüllende Voraussetzung, es wurde aber überraschender Weise festgestellt, dass das Entkeimungsmittel enthaltene Behandlungsmittel innerhalb der Dauer der Aussetzung vollständig diffundiert und die verbleibenden Rückstände auch auf relativ glatten Oberflächen unterhalb der gesetzlich erlaubten Menge sinken. Das Behandlungsmittel verbleibt also sozusagen auf den behandelten Gebrauchsgüter, verringert sich jedoch in seiner Menge und seiner Wirkung innerhalb der angegebenen Dauer so sehr, dass die gesetzlichen Vorgaben hinsichtlich der Kontaminierung von Behandlungsmitteln mit Lebensmitteln in Kontakt tretenden Oberflächen von Gebrauchsgütern bei Weitem erfüllt werden können.

Von großem Vorteil ist es, wenn das Behandlungsmittel frei von einem Reaktionsbeschleuniger ist. Obwohl es zunächst widersinnig scheint, der geringen Menge des zur Anwendung kommenden Entkeimungsmittels keinen wirksamen Reaktionsbeschleuniger, wie es beispielsweise Ameisensäure für H₂O₂ bildet, enthalten zu wollen. Es hat sich jedoch überraschender Weise gezeigt, dass die sich ausbildende Atmosphäre ausreichend ist, um die ihr ausgesetzten Gebrauchsgüter sicher zu sterilisieren, auch wenn auf einen Reaktionsbeschleuniger verzichtet wird. Zudem ist die Wirkungsdauer der sterilisierenden Atmosphähre auch über einen längeren Zeitraum möglich. Reaktionsbeschleuniger sind nämlich instabil und haben im höchsten Fall eine Einsatzfähigkeit von 12 Stunden. Treten das Entkeimungsmittel und der Reaktionsbeschleuniger im Behandlungsmittel oder auch erst in der sich ausbildenden sterilisierenden Atmosphäre in Kontakt ist mit dem Reaktionsbeschleuniger auch die Wirksamkeitsdauer des Entkeimungsmittels auf die Wirksamkeitsdauer des Reaktionsbeschleunigers begrenzt. Im Beispiel, dass das Entkeimungsmittel durch H₂O₂ oder eine wässrige Lösung davon gebildet wird und der Reaktionsbeschleuniger Ameisensäure enthält oder aus Ameisensäure gebildet ist, ist die tatsächliche Wirksamkeitsdauer gemeinsam auf maximal 12 Stunden begrenzt. Dagegen hat sich gezeigt, dass die die Reduktionsfähigkeit der sich auf die Gebrauchsgüter niedergeschlagenen sterilisierenden Atmosphäre bei Verzicht auf einen solchen Reaktionsbeschleuniger sehr viel besser ist.

Eine weitere Ausgestaltung der Erfindung sieht vor, dass ein Behandlungsmittel verwendet wird, welches einen Farbstoff enthält und eine Verfärbung der behandelten Sammelbehälter bewirkt, so dass optisch erkennbar und ggf. maschinell überprüfbar ist, dass alle gefüllten und behandelten Sammelbehälter mit Behandlungsmittel beladen sind.

Eine weitere Ausgestaltung der Erfindung sieht vor, dass die bestimmte Menge an Gebrauchsgütern in Einzelteilen und/oder einzelnen Baugruppen gemessen wird und dass die Menge an Einzelteilen und/oder einzelnen Baugruppen ≥ 100, insbesondere ≥500, ganz insbesondere ≥ 800 ist.

Auf diese Weise kann das Verfahren besonders effektiv angewendet werden. Überraschenderweise hat sich dabei gezeigt, dass die Einzelteile, insbesondere die einzelnen Öffnungs- und/oder Verschlusselemente, auch in den genannten Größenbereichen absolut zuverlässig sterilisiert werden. Tests haben sogar ergeben, dass die Anzahl der Einzelteile noch größer sein kann. Bezogen auf ein Volumen des Sammelbehälters von etwa 56 Litern können sogar 1.200 bis 1.500 Einzelteile, insbesondere einzelne Öffnungs- und/oder Verschlusselemente, wie sie heute für Lebensmittelverpackungen, insbesondere aseptisch hergestellten Getränkeverpackungen, mit Packungsvolumina zwischen etwa 100 ml und etwa 2.000 ml üblich sind sterilisiert werden.

Um eine hohe Produktivität erreichen zu können, ist vorgesehen, dass das Verfahren frei von einem Positionieren des Gebrauchsguts oder der Gebrauchsgüter ist. Die Gebrauchsgüter werden also beispielsweise gemeinsam als Schüttgut in den Sammelbehälter eingefüllt oder gelangen unmittelbar oder mittelbar am Ausgang einer Produktionsmaschine in den Sammelbehälter.

Um zu verhindern, dass im Inneren des Sammelbehälters eine Kondensation von Beladungsmittel stattfindet, ist nach einer weiteren Ausgestaltung der Erfindung vorgesehen, dass das Kunststoffmaterial des Sammelbehälters eine vorgegebene Permeabilität aufweist.

Eine weitere Lehre der Erfindung sieht vor, dass als Sammelbehälter Kunststoffbehälter aus Polyethylen (PE) bzw. Polyamid (PA) verwendet werden.

An Stelle des Besprühens der Gebrauchsgüter ist es in einer weiteren Ausbildung der Erfindung auch möglich, dass ein Beladungsträger, beispielsweise ein Streifen aus Filz oder dergleichen, welcher mit der ausreichenden Menge Sterilisationsmittel behandelt worden ist, in den Sammelbehälter eingebracht werden kann.

Weiterhin ist es möglich, auch die verwendete Umverpackung in ihrem Inneren teilweise noch mit Beladungsmittel zu benetzen, um zuverlässig auszuschließen, dass sich im Inneren des - geschlossenen - Umkartons aus dessen Material keimbildende Sporen in den oder die Sammelbehälter gelangen.

Schließlich ist es auch möglich, die Umverpackung in ihrem Inneren mit einem oder mehreren Beladungsträgern zu versehen, um die gewünschte Langzeitsterilisation während des Transports zu erreichen. Die Beladungsträger können dazu beispielsweise in die Umkartons eingelegt oder eingeklebt werden.

Die Erfindung wird nachfolgend anhand einer lediglich bevorzugte Ausführungsbeispiele darstellenden Zeichnung näher erläutert. In der Zeichnung zeigen
- Fig. 1: einen leeren Sammelbehälter in einer Umverpackung in perspektivischer Darstellung,
- Fig. 2: den gefüllten Sammelbehälter aus Fig.1,
- Fig. 3: den verschlossenen Sammelbehälter aus Fig. 2 in offener Umverpackung,
- Fig. 4: die geschlossene Umverpackung und
- Fig. 5: ein weiteres Ausführungsbeispiel hinsichtlich des Einbringens des Sterilisationsmittels.

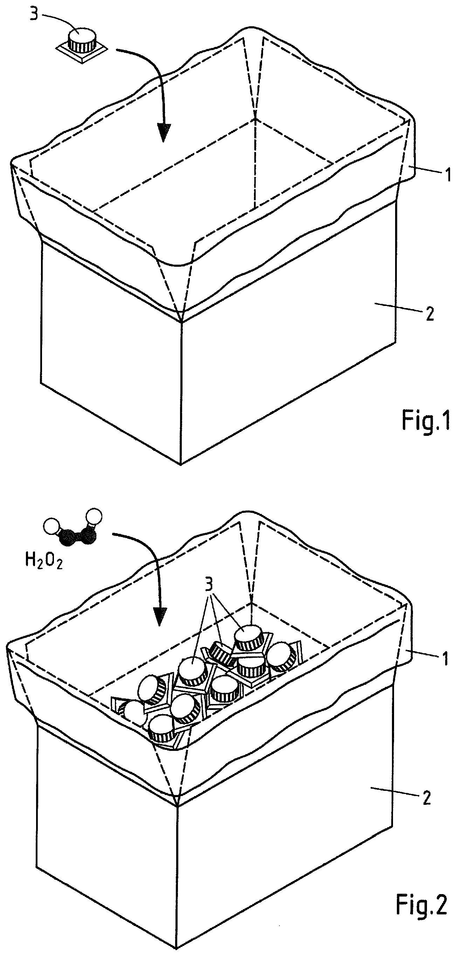

Fig. 1 zeigt einen als Kunststoff-Beutel ausgeführten Sammelbehälter 1, welcher derart in einen offenen Umkarton 2 eingelegt ist, dass sein oberer Rand über die Laschen des Umkartons 2 geführt werden kann. Auf diese Weise kann zuverlässig vermieden werden, dass ein Kontakt zwischen den in den Sammelbehälter 1 einzufüllenden Gebrauchsgüter 3 und dem Umkarton vermieden wird. Als Gebrauchsgüter werden beispielhaft Öffnungs- und/oder Verschlusselemente zur späteren Verwendung mit Getränke- bzw. Lebensmittelpackungen dargestellt.

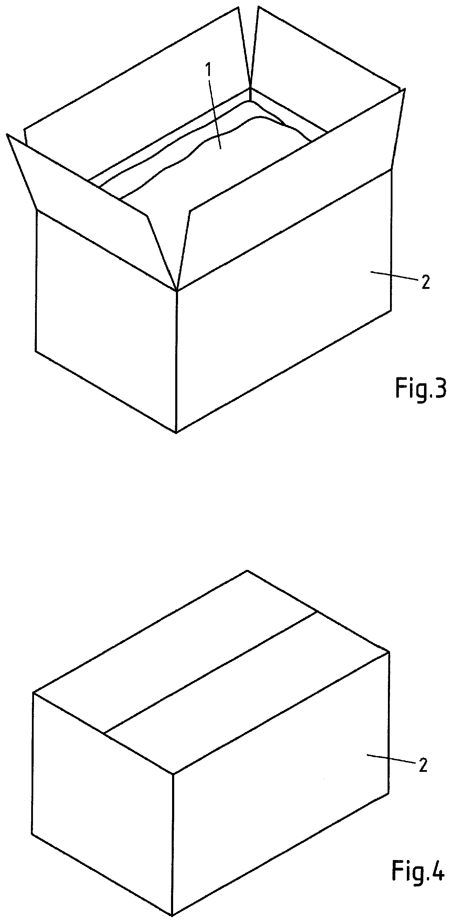

In Fig. 2 sieht man nun einen mit den vorgenannten Gebrauchsgütern 3 gefüllten Sammelbehälter 1, in den zur Sterilisation eine vorgegebene Menge des Behandlungsmittels H₂O₂ eingebracht wird. Anschließend wird der Behälter 1 verschlossen, wie in Fig. 3 dargestellt und der Umkarton 2 schließlich verschlossen, wie in Fig. 4 dargestellt ist.

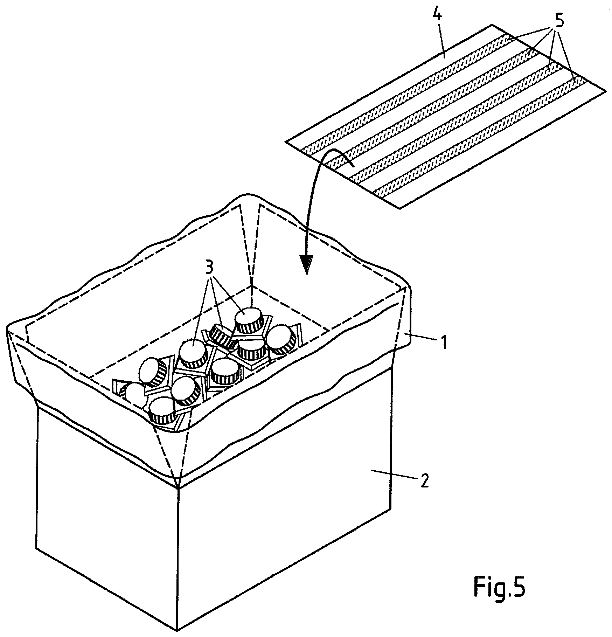

Schließlich zeigt Fig. 5 eine weitere Möglichkeit des Einbringens des Sterilisationsmittels, bei dem dieses mittels eines Einlageblattes 4 erfolgt, auf das verschiedene Beladungsträger 5, welche mit einem Sterilisationsmittel beladen sind, aufgebracht wurden.

## Patentansprüche

1. Verfahren zum Sterilisieren von unverpackten Gebrauchsgütern, wie insbesondere aus Kunststoff bestehenden Öffnungs- und/oder Verschlusselementen zur späteren Verwendung mit Getränke- bzw. Lebensmittelpackungen, mittels einem ein Entkeimungsmittel aufweisenden Behandlungsmittel, wobei eine bestimmte Menge an unverpackten Gebrauchsgütern (3) gemeinsam für eine vorgegebene Zeit in einem Sammelbehälter (1) gelagert und das Innere des Sammelbehälters (1) mit einer vorgegebenen Menge des Behandlungsmittels beladen wird,
**dadurch gekennzeichnet, dass**
ein Beladungsträger, welcher mit Sterilisationsmittel behandelt worden ist, in den Sammelbehälter (1) eingebracht wird.

2. Verfahren nach Anspruch 1,
**dadurch gekennzeichnet, dass**
die Beladung maschinell erfolgt, vorzugsweise mittels einer Sprühdüse.

3. Verfahren nach Anspruch 1,
**dadurch gekennzeichnet, dass**
die Beladung manuell erfolgt.

4. Verfahren nach einem der Ansprüche 1 bis 3,
**dadurch gekennzeichnet, dass**
als Beladungsmittel Wasserstoffperoxid (H₂O₂) verwendet wird und dass die Konzentration des Behandlungsmittels zwischen 20 % und 50 % beträgt.

5. Verfahren nach Anspruch 4,
**dadurch gekennzeichnet, dass**
die Konzentration des Behandlungsmittels 35 % beträgt.

6. Verfahren nach einem der Ansprüche 1 bis 5,
**dadurch gekennzeichnet, dass**
die Menge der jeweiligen Beladung mit Beladungsmittel in Abhängigkeit vom Volumen und von der Größe bzw. Oberfläche der zu sterilisierenden Gebrauchsgüter (3) zwischen 0,5 ml und 2 ml beträgt.

7. Verfahren nach einem der Ansprüche 1 bis 6,
**dadurch gekennzeichnet, dass**
die Dauer der Aussetzung mit Beladungsmittel im Sammelbehälter (1) zwischen 12 Stunden und 21 Tagen beträgt.

8. Verfahren nach Anspruch 7,
**dadurch gekennzeichnet, dass**
die Dauer der Aussetzung mit Beladungsmittel im Sammelbehälter (1) zwischen 24 Stunden und 7 Tagen beträgt.

9. Verfahren nach einem der Ansprüche 1 bis 8,
**dadurch gekennzeichnet, dass**,
das Verfahren frei von einer aktiven Entfernung des Behandlungsmittels ist.

10. Verfahren nach einem der Ansprüche 1 bis 9,
**dadurch gekennzeichnet, dass**,
das Behandlungsmittel frei von einem Reaktionsbeschleuniger ist.

11. Verfahren nach einem der Ansprüche 1 bis 10,
**dadurch gekennzeichnet, dass**
ein Behandlungsmittel verwendet wird, welches einen Farbstoff enthält und eine Verfärbung der behandelten Sammelbehälter (1) bewirkt.

12. Verfahren nach einem der Ansprüche 1 bis 11,
**dadurch gekennzeichnet, dass**
die bestimmte Menge an Gebrauchsgütern in Einzelteilen und/oder einzelnen Baugruppen gemessen wird und dass die Menge an Einzelteilen und/oder einzelnen Baugruppen ≥100, insbesondere ≥ 500, ganz insbesondere ≥ 800 ist.

13. Verfahren nach einem der Ansprüche 1 bis 12,
**dadurch gekennzeichnet, dass**
das Verfahren frei von einem Positionieren des oder der Gebrauchsgüter ist.

14. Sammelbehälter zur Aufnahme einer Vielzahl ungeordneter unverpackter Gebrauchsgüter, wie insbesondere aus Kunststoff bestehenden Öffnungs- und/oder Verschlusselementen, zur Verwendung mit einem Verfahren nach den Ansprüchen 1 bis 13, wobei das Material für den Sammelbehälter (1) Kunststoff ist und der Sammelbehälter (1) als Beutel ausgeführt ist, welcher nach der Befüllung mit unverpackten Gebrauchsgütern (3) und anschließender Beladung verschlossen und in einer anschließend verschlossenen Umverpackung gelagert wird,
**dadurch gekennzeichnet, dass**
ein Beladungsträger, der mit der ausreichenden Menge Sterilisationsmittel behandelt worden ist, in den Sammelbehälter (1) eingebracht wird.

15. Sammelbehälter nach Anspruch 14,
**dadurch gekennzeichnet, dass**
eine eingestellte Permeabilität im Kunststoffmaterial eine Kondensation von Beladungsmittel im Inneren des Sammelbehälters (1) verhindert.

16. Sammelbehälter nach Anspruch 15,
**dadurch gekennzeichnet, dass**
als Sammelbehälter (1) ein Kunststoffbeutel aus Polyethylen (PE) vorgesehen ist.

17. Sammelbehälter nach Anspruch 15,
**dadurch gekennzeichnet, dass**
als Sammelbehälter (1) ein Kunststoffbeutel aus Polyamid (PA) vorgesehen ist.

18. Sammelbehälter nach einem der Ansprüche 14 bis 17,
**dadurch gekennzeichnet, dass**
der Beutel zum Verschließen an seiner offenen Seite eingeschlagen (gefaltet) wird.

19. Umverpackung zur Aufnahme eines oder mehrerer Sammelbehälter/s zur Verwendung mit einem Verfahren nach den Ansprüchen 1 bis 13, wobei als Material für die Umverpackung Karton, insbesondere Wellpappe, verwendet wird, so dass ein Umkarton (2) mit schließbaren Laschen entsteht, welche zum Verschließen des Umkartons (2) umgelegt und entsprechend fixiert werden,
**dadurch gekennzeichnet, dass**
der Umkarton (2) in seinem Inneren teilweise mit Beladungsmittel benetzt wird oder ein Beladungsträger, der mit der ausreichenden Menge Sterilisationsmittel behandelt worden ist, in den Umkarton (2) eingebracht wird.

## Claims

1. A method for sterilising packaged or partially packaged or partially sealed consumable items, such as in particular plastic opening and/or closing elements for subsequent use with drink or food packaging, by means of a treatment agent having a disinfectant, wherein a plurality of unpackaged consumable items (3) are stored together for a specified time in a container (1) and in that the inside of the container (1) is treated with a predetermined quantity of the treatment agent, **characterised in that** a treatment carrier, treated with sterilisation agent, being introduced into the container (1).

2. A method according to Claim 1, **characterised in that** the treatment is performed mechanically, preferably by means of a spray nozzle.

3. A method according to Claim 1, **characterised in that** the treatment is performed manually.

4. A method according to any one of Claims 1 to 3, **characterised in that** as the treatment agent hydrogen peroxide (H₂O₂) is used and **in that** the concentration of the treatment agent is between 20 % and 50 %.

5. A method according to Claim 4, **characterised in that** the concentration of the treatment agent is 35 %.

6. A method according to any one of Claims 1 to 5, **characterised in that** the amount of the respective treatment with treatment agent is, dependent upon the volume and the size of the surface of the consumable items (3), between 0.5 ml and 2 ml.

7. A method according to any one of Claims 1 to 6, **characterised in that** the duration of the exposure to treatment agent in the container (1) is between 12 hours and 21 days.

8. A method according to Claim 7, **characterised in that** the duration of the exposure to treatment agent in the container (1) is between 24 hours and 7 days.

9. A method according to any one of Claims 1 to 8, **characterised in that** the method is free from active removal of the treatment agent.

10. A method according to any one of Claims 1 to 9, **characterised in that** the treatment agent used is free from a reaction accelerator.

11. A method according to any one of Claims 1 to 10, **characterised in that** a treatment agent is used containing a dye and brings about a discoloration of the container (1).

12. A method according to any one of Claims 1 to 11, **characterised in that** the specified quantity of consumable items is measured in individual parts and/or individual modules and that the quantity of individual parts and/or individual modules is ≥100, in particular ≥ 500, quite particularly ≥ 800.

13. A method according to any one of Claims 1 to 12, **characterised in that** the method is free from positioning of the consumable item or consumable items.

14. A container for accommodating a plurality of unarranged unpackaged consumable items, such as in particular plastic opening and/or closing elements for use with a method according to Claims 1 to 13, wherein the material for the container (1) is plastic, wherein the container (1) is designed as a bag which after filling with unpackaged consumable items (3) and subsequent treatment is sealed and stored in a subsequently sealed outer packaging, **characterised in that** and **in that** a treatment carrier, treated with an appropriate quantity of sterilisation agent, is introduced into the container (1).

15. A container according to Claim 14, **characterised in that** the plastic material has a set permeability so that condensation of treatment agent inside the container (1) is prevented.

16. A container according to Claim 15, **characterised in that** as the container (1) a plastic bag in polyethylene (PE) is provided.

17. A container according to Claim 15, **characterised in that** as the container (1) a plastic bag in polyamide (PA) is provided.

18. A container according to any one of Claims 14 to 17, **characterised in that** the bag is sealed at its open end (folded).

19. An outer packaging for accommodating one or more container(s) for use with a method according to Claims 1 to 13, wherein as material for the outer packaging cardboard, in particular corrugated cardboard, is used, so that an outer carton (2) with closable flaps results, which are folded down and suitably secured in order to close the outer carton (2), **characterised in that** the outer carton (2) is partially wetted on its inside with treatment agent or a treatment carrier, treated with a suitable quantity of sterilisation agent, is introduced into the outer carton (2).

## Revendications

1. Procédé de stérilisation de produits de consommation non emballés, comme, en particulier, des éléments d'ouverture et / ou de fermeture consistant en matière synthétique, destinés à être utilisés plus tard en commun avec des emballages de boissons ou respectivement de produits alimentaires, au moyen d'un produit de traitement contenant un agent désinfectant, sachant qu'une quantité déterminée de produits de consommation non emballés (3) est stockée pendant un temps prédéterminé dans un récipient collecteur (1) et que l'intérieur du récipient collecteur (1) est chargé avec une quantité prédéterminée de produit de traitement,
**caractérisé en ce que**,
dans le récipient collecteur (1), se trouve un support de chargement, qui est traité avec un produit de stérilisation.

2. Procédé selon la revendication 1,
**caractérisé en ce que**
le chargement est effectué mécaniquement, de préférence au moyen d'une buse de pulvérisation.

3. Procédé selon la revendication 1,
**caractérisé en ce que**
le chargement est effectué manuellement.

4. Procédé selon l'une des revendications 1 à 3,
**caractérisé en ce que**,
comme produit de chargement, on utilise du peroxyde d'hydrogène (H₂O₂) et que la concentration du produit de traitement est située entre 20 % et 50 %.

5. Procédé selon la revendication 4,
**caractérisé en ce que**
la concentration du produit de traitement est de 35 %.

6. Procédé selon l'une des revendications 1 à 5,
**caractérise en ce que**
la quantité de chaque chargement de produit de chargement, en fonction du volume et de la grandeur, respectivement de la surface des produits de consommation (3) à stériliser, est située entre 0,5 ml et 2 ml.

7. Procédé selon l'une des revendications 1 à 6,
**caractérise en ce que**
la durée de l'exposition aux produits de chargement dans le récipient collecteur (1) est située entre 12 heures et 21 jours.

8. Procédé selon la revendication 7,
**caractérisé en ce que**
la durée de l'exposition aux produits de chargement dans le récipient collecteur (1) est située entre 24 heures et 7 jours.

9. Procédé selon l'une des revendications 1 à 8,
**caractérisé en ce que**
le procédé est exempt d'enlèvement actif du produit de traitement.

10. Procédé selon l'une des revendications 1 à 9,
**caractérisé en ce que**
le produit de traitement est exempt d'accélérateur de réaction.

11. Procédé selon l'une des revendications 1 à 10,
**caractérisé en ce que**
l'on utilise un produit de traitement qui contient une matière colorante et qui provoque une coloration du récipient collecteur (1) traité.

12. Procédé selon l'une des revendications 1 à 11,
**caractérisé en ce que**
la quantité déterminée de produits de consommation est mesurée en pièces individuelles et / ou en groupes de composants et que la quantité de pièces individuelles et / ou de groupes de composants est ≥ 100, en particulier ≥ 500, tout particulièrement ≥ 800.

13. Procédé selon l'une des revendications 1 à 12,
**caractérisé en ce que**
le procédé est exempt de positionnement du ou des produit/s de consommation.

14. Récipient collecteur pout la réception d'une pluralité de produits de consommation en vrac, non emballés, comme, en particulier, des éléments d'ouverture et / ou de fermeture consistant en matière synthétique, lequel est destiné à être utilisé avec un procédé selon l'une des revendications 1 à 13, sachant que le matériau utilisé pour le récipient collecteur (1) est un matériau synthétique, et que le récipient collecteur (1) est réalisé en forme de sac, lequel est fermé après le remplissage avec des produits de consommation non emballés (3) et le chargement y faisant suit, et est ensuite stocké dans un emballage fermé,
**caractérisé en ce que**,
dans le récipient collecteur (1), est placé un support de chargement qui a été traité avec la quantité de produit de stérilisation suffisante.

15. Récipient collecteur selon la revendication 14,
**caractérisé en ce que**
la perméabilité réglée dans le matériau synthétique empêche une condensation du produit de chargement à l'intérieure du récipient collecteur (1).

16. Récipient collecteur selon la revendication 15,
**caractérisé en ce que**,
comme récipient collecteur (1), est prévu un sac plastique en polyéthylène (PE).

17. Récipient collecteur selon la revendication 15,
**caractérisé en ce que**,
comme récipient collecteur (1), est prévu un sac plastique en polyamide (PA).

18. Récipient collecteur selon l'une des revendications 14 à 17,
**caractérisé en ce que**,
pour la fermeture, le sac est rabattu (plié) sur son côté ouvert.

19. Emballage destiné à recevoir un ou plusieurs récipient/s collecteur/s pour l'utilisation avec un procédé selon les revendications 1 à 13, sachant que, pour l'emballage, on utilise, en tant que matière, du carton, en particulier du carton ondulé, de manière à obtenir un carton d'emballage (2) pourvu de brides, qui, pouvant être fermées, sont rabattues pour fermer le carton et fixées adéquatement,
**caractérisé en ce que**
le carton d'emballage (2) est partiellement humecté, à l'intérieur, avec du produit de chargement ou qu'un support de chargement, qui a été traité avec la quantité suffisante de produit de stérilisation, est placé dans le carton d'emballage (2).
